(19) Europäisches Patentamt
European Patent Office
Office européen des brevets

(11) Veröffentlichungsnummer: **0 237 880**
**B1**

(12) # EUROPÄISCHE PATENTSCHRIFT

(45) Veröffentlichungstag der Patentschrift: **09.01.91**

(51) Int. Cl.$^5$: **A 01 N 25/02**

(21) Anmeldenummer: **87103149.8**

(22) Anmeldetag: **05.03.87**

(54) **Phospholipidhaltige Produkte, deren Herstellung und Verwendung.**

(30) Priorität: **14.03.86 DE 3608455**

(43) Veröffentlichungstag der Anmeldung:
**23.09.87 Patentblatt 87/39**

(45) Bekanntmachung des Hinweises auf die Patenterteilung:
**09.01.91 Patentblatt 91/02**

(84) Benannte Vertragsstaaten:
**AT BE CH DE ES FR GB GR IT LI LU NL SE**

(56) Entgegenhaltungen:
**EP-A-0 068 295**
**AU-A- 498 895**
**DE-A-2 715 286**
**DE-A-3 218 027**
**DE-B-2 422 449**
**US-A-1 938 864**
**US-A-2 258 832**

(73) Patentinhaber: **A. Nattermann & Cie. GmbH**
**Nattermannallee 1**
**D-5000 Köln 30 (DE)**

(72) Erfinder: **Hager, Jörg**
**Herm. Jos. Schmidt-Strasse 48**
**D-5000 Köln 30 (DE)**
Erfinder: **Ghyczy, Miklos, Dr.**
**Im Kapsfeld 23**
**D-5000 Köln 41 (DE)**
Erfinder: **Feyen, Vincent, Dr.**
**Rote Kreuz-Strasse 57**
**D-5010 Bergheim 3 (DE)**
Erfinder: **Imberge, Paul**
**Kiefernweg 5**
**D-5024 Pulheim-Sinthern (DE)**
Erfinder: **Brandenburg, Ulrich**
**Akazienweg 57**
**D-5000 Köln 40 (DE)**
Erfinder: **Wilperath, Peter**
**August Kierspel-Strasse 161**
**D-5060 Bergisch Gladbach 2 (DE)**

(74) Vertreter: **Sternagel, Hans-Günther, Dr. et al**
**Patentanwälte Dr. Michael Hann Dr. H.-G. Sternagel Sander Aue 30**
**D-5060 Bergisch Gladbach 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 237 880 B1

**Beschreibung**

Lecithine bzw. Phospholipide spielen sowohl in der Lebensmittelindustrie als auch in der Chemischen und Pharmazeutischen Industrie eine große Rolle, weil sie hervorragende physiologische Eigenschaften aufweisen und außerdem mit ihrer Hilfe viele Produkte physikalisch verbessert werden können. Das ist u.a. auf emulgierende, weichmachende, kolloidale, antioxidative und oberflächenaktive Effekte zurückzuführen. Die wichtigsten Lecithinverarbeiter sind: Öl- und Margarineindustrie, Futtermittelindustrie, Süßwarenindustrie, Farbenindustrie, Backwarenindustrie, Mineralölindustrie, Lederindustrie, Textilindustrie, Gummiindustrie, Pflanzenschutzmittelindustrie, Pharmazeutische Industrie, Kosmetikindustrie und Seifenindustrie. Lecithin besitzt bei vielen Herstellungsverfahren eine kombinierte Wirkung, aus der sich seine Überlegenheit gegenüber synthetischen Stoffen ergibt. Darüber hinaus ist es ernährungsphysiologisch unbedenklich und deshalb für die Verwendung in der Lebensmittelindustrie geeignet.

Die Phospholipide kommen in der Natur in großen Umfang vor und können aus tierischen und pflanzlichen Materialien gewonnen werden. Hauptquellen sind Eier (Ei-Lecithin), Ölsaaten und Ölfrüchte, wie z.B. Kokosnuß-Kopra, Palmkerne, Erdnüsse, Raps, Sonnenblumenkerne, Sojabohnen, Ölpalmen und Oliven. Die Phospholipide fallen vorwiegend bei der Gewinnung der pflanzlichen Öle als Nebenprodukt an.

Die Gewinnung der pflanzlichen Öle erfolgt entweder durch Auspressen oder durch Extraktion mit Fettlösungsmitteln. Häufig werden auch beide Verfahren angewendet, indem man zunächst auspreßt und anschließend die dabei enthaltenen Preßrückstände extrahiert. Der Preßrückstand bzw. der Extraktionsschrot ist mengenmäßig das wichtigste Nebenprodukt bei der Ölgewinnung. Der Preßrückstand bzw. der Extraktionsschrot ist wegen seines hohen Proteingehaltes ein begehrtes Kraftfutter für die Landwirtschaft.

Die durch Abpressen oder durch Extraktion erhaltenen Fette und Öle müssen, wenn sie Nahrungsmittelzwecken dienen, einer weitgehenden Reinigung unterzogen werden. Diese Reinigung wird auch als Raffination bezeichnet. Einer der wichtigsten Reinigungsschritte ist die sogenannte Entschleimung, wobei unterwünschte Phospholipide, die zusammen mit dem Neutralöl aus den Ölsaaten herausgelöst werden, aus dem Rohöl entfernt werden, um die Stabilität und Lagerfähigkeit der Öle zu erhöhen.

Die Entschleimung erfolgt durch Einleiten von geringen Mengen Wasserdampf oder Wasser bei höheren Temperaturen in das Rohöl. Dabei bildet sich eine viskose Masse, der sogenannte Lecithinschlamm. Diese Lecithinschlämme haben je nach Herkunft unterschiedliche Zusammensetzung:

14—36 Gew.-% Pflanzenöl
27—56 Gew.-% Wasser
58— 8 Gew.-% Phospholipide.

Dieses Nebenprodukt bei der Ölgewinnung wird entweder direkt wieder aus dem Schrot versprüht und als Futtermittel verwendet, oder in einem Verdampfer bei höheren Temperaturen (ca. 80°C) über eine längere Zeit (ab 6—12 Std.) oder bei 100°C im Dünnschichtverdampfer mit kürzerer Verweilzeit bis auf einen Restwassergehalt von 0,5—2% eingedampft. Durch diese Trocknung des Lecithinschlammes erhält man das handelsübliche Rohlecithin. Wichtigstes Rohlecithin ist das Sojalecithin, welches nach der Trocknung ca.

52 Gew.-% Phospholipide
35 Gew.-% Öle und Fettsäuren
10 Gew.-% Glykolipid und Zucker
2 Gew.-% unverseifbare Bestandteile und
1 Gew.-% Wasser

Durch die Behandlung mit entsprechenden Lösungsmitteln, z.B. mit Aceton, kommt man zu sogenannten entölten Phospholipiden (bzw. entöltem Rohlecithin, welches nur noch geringe Mengen an Öl und sonstigen Begleitlipiden enthält).

Die gewonnenen Lecithinfraktionen haben je nach Herkunft unterschiedliche Phospholipid-Zusammensetzung:

Soja-Lecithin: ca. 30% Phosphatidylcholin, 1—2% Lysophosphatidylcholin, 22% Phosphatidylethanolamin, 1—2% Lysophosphatidylethanolamin, 3—4% Phosphatidylserin, 18% Phosphatidylinosit, 13% Phytoglykolipide, 2% Phosphatidsäure, 8% Begleitlipide.

Ei-Lecithin: 73% Phosphatidylcholin, 5—6% Lysophosphatidylcholin, 15% Phosphatidylethanolamin, 2—3% Lysophosphatidylethanolamin, 1% Phosphatidylinosit, 2—3% Sphingomyelin, 1% Plasmalogen.

Raps-Lecithin: 30—32% Phosphatidylcholin, 3% Lysophosphatidylcholin, 30—32% Phosphatidylcholin, 3% Lysophosphatidylethanolamin, 14—18% Phosphatidylinosit, 1% Lysophosphatidylinosit, 10% Phytoglykolipide, 1% Phosphatidsäure, 2—3% Begleitlipide.

Saflor-Lecithin: 32—39% Phosphatidylcholin, 1—2% Lysophosphatidylcholin, 14—17% Phosphatidylethanolamin, 2% Lysophosphatidylethanolamin, 21—27% Phosphatidylinosit, 1% Lysophosphatidylinosit, 15—28% Begleitlipide.

Die einzelnen Lecithine können auch nach bekannten Verfahren gereinigt werden und die entsprechenden Phospholipide in die einzelnen Bestandteile, wie Phosphatidylcholin, Phosphatidylethanolamin, Phosphatidylinosit, Phosphatidylserin, Phosphatidylglycerol,

Lysophosphatidylcholin, Lysophosphatidylethanolamin, Lysophosphatidylserin, Lysophosphatidylglycerol, aufgetrennt werden oder auch olefinische Gemische hergestellt werden.

So sind z.B. reine Phospholipid-Produkte im Handel, die z.B. folgende Zusammensetzung haben können (EP 68295).

| | |
|---|---|
| Phospholipon$^R$ 25 | 25% Phosphatidylcholin<br>25% Phosphatidylethanolamin<br>20% Phosphatidylinosit |
| Phospholipon$^R$ 55 | 55% Phosphatidylcholin<br>25% Phosphatidylethanolamin<br>2% Phosphatidylinosit |
| Phospholipon$^R$ 80 | 80% Phosphatidylcholin<br>10% Phosphatidylethanolamin |
| Phospholipon$^R$ 100 | 96% Phosphatidylcholin |
| Phospholipon$^R$ 100 H | 96% Phosphatidylcholin hydriert |
| Phospholipon$^R$ 38 | 38% Phosphatidylcholin<br>16% N-Acetyl-phosphatidylethanolamin<br>4% Phosphatidylethanolamin |

Nun besitzen die sehr unterschiedlich zusammengesetzten Phospholipidgemische, angefangen vom Lecithinnaßschlamm, Rohlecithin, entöltem Lecithin bis zu definierten zusammengesetzten Phospholipidgemischen oder sogar den reinen Phospholipiden, wie z.B. Phosphatidylcholin, sehr stark voneinander abweichende physikalische Eigenschaften. Diese Phospholipidgemische besitzen eine sehr unterschiedliche Konsistenz von flüssig bis zähplastisch.

Um diese Gemische einer Anwendung zuführen zu können, müssen sie durch entsprechende Maßnahmen unter Zusatz von z.B. Emulgatoren, Lösungsmitteln, Verflüssigern und dergl. in der entsprechende verarbeitbare Form gebracht werden. Für viele Anwendungsgebiete ist es wünschenswert, die wasserunlöslichen Phospholipidgemische in Wasser lösen bzw. emulgieren zu können. Es gibt bereits viele Versuche unterschiedlichlicher Phospholipidgemische bzw. Phospholipidhaltige Gemische in Wasser zu lössen bzw. zu emulgieren, in EP—A—98561 werden z.B. organische Lösungsmittel und Emulgatoren zugesetzt. In DE—B—1141639 wird reines Phosphatidylcholin durch Zusatz von Gallensäuren wasserlöslich gemacht. In DE—A—1227191 werden Lecithine mit aliphatischen Polyalkohlen in Gegenwart von Ethanol in Wasser emulgiert. In DE—A—1617542 wird entöltes Rohlecithin in wäßrige zuckerhaltige Alkohole wasserlöslich gemacht. Ölhaltige Lecithine können nach US—A—2402690 durch Zusatz von Monoglyceriden wasserdispergierbar gemacht werden. In DE—B 3218027 wird eine Verflüssigung und Wasserlöslichkeit von Phospholipiden durch Zusatz von Hydroxyethylfethosäureamide erhalten. Alle bisherigen Verfahren haben den Nachteil, daß sie jeweils individuell für ein bestimmtes Phospholipid oder Lecithingemisch entwickelt wurden und deshalb bei Übertragung auf ein anderes Gemisch, z.B. Lecithinnaßschlamm, versagen.

Ziel der vorliegenden Erfindung war es deshalb, einen Zusatz und eine Methode zu finden, bei der Phospholipidgemische mit unterschiedlichster Konzentration und Zusammensetzung in Wasser gelöst, emulgiert oder dispergiert werden können.

Diese Aufgabe wird gelöst durch wässrige Phospholipidlösungen, die Lösungsvermittler enthalten, dadurch gekennzeichnet, daß als Lösungsvermittler ein oder mehrere Verbindungen der Formel (I)

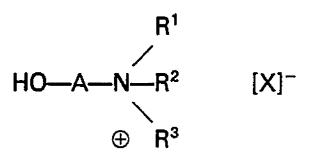

I

in der A eine gradkettige Alkylengruppe mit 2 bis 4 Kohlenstoffatomen, $R^1$, $R^2$, $R^3$ gleich oder verschieden sein können und Wasserstoff oder Methyl bedeuten und X das zur Salzbildung dienende Anion einer anorganischen oder organischen Säure ist, und/oder 1-Methylamino-2-hydroxy-ethan, 1-Dimethylamino-2-hydroxyethan, 1-Dimethylamino-2-hydroxy-butan, 1-Hydroxy-2-methylamino-hexan, 1-Amino-2-hydroxy-ethan, 1-Amino-3-hydroxy-propan, 1-Amino-2-hydroxy-propan, 1-Dimethylamino-2-hydroxy-propan, 1-Amino-3-hydroxy-butan vorhanden sind.

Biespiele für Verbindungen der Formel 1 sind zum Beispiel:

Salze von

1-Trimethylamino-2-hydroxy-propan

1-Hydroxy-2-trimethylamino-ethan-hydrochlorid

Salze von

1-Trimethylamino-3-hydroxy-propan und

4-Hydroxy-1-trimethylamino-butan

1-Trimethylamino-1-hydroxy-propan-hydrochlorid

Besonders bevorzugt sind 1-Amino-2-hydroxy-ethan und 1-Hydroxy-2-trimethylamino-ethan und deren Salze, die einzeln oder im Gemisch in Konzentrationen 3—10 Gew.-% zu der zu lösenden Phospholipidmischung gegeben werden. Die Menge von 10 Gew.-% kann ggf. auch überschritten werden. Bevorzugt werden jedoch 5—7 Gew.-% der Verbindung oder Gemische davon zugesetzt, besonders bevorzugt ist der Zusatz von 6 Gew.-%. Besonders vorteilhaft sind Mischungen aus 1-Amino-2-hydroxy-alkanen und 1-Hydroxy-2-trimethyl-amino-alkane, wobei 0,8—1,2 Gew.-% des Aminohydroxyalkans und 4,8—5,2 Gew.-% Trimethylaminohydroxyalkansalz zusammen eingesetzt werden. Zusätzlich können ggf. weitere übliche Hilfsstoffe zugesetzt werden, wie z.B. Konservierungsmittel und Emulgatoren. Als Konservierungsmittel kommen z.B. Formalinlösungen, Preventol® D 3, Benzoesäure oder Sorbinsäure in Frage.

Als Emulgatoren können z.B. Fettalkoholethoxylate (z.B. die Handelsprodukte Merlipal®, Lorox®, Steinapal®, Emulgien®), ethoxylierte Fettamine, (z.B. die Handelsprodukte Ethomeen®, Genamin®, Araphen®), Alkylphenolethoxylate (z.B. die Handelsprodukte Antarox®, Atlas-Renex-Produkte), Nonylphenolpolyglykolether, ethoxylierte Fettsäureester, Sorbitanfettsäureester, ethoxylierte Sorbitanfettsäureester, Betaine, Kokosamin-ethoxyliert, Alkylpolyglykolether, Polyoxyethylen-(20)-Sorbitanmonolaurat, Polyoxyalkylen-(20)-Sorbitanmonopalmitat, Glycerin-Polyethylenglykoloxystearat, Capronsäurehydroxyethylamid, Alkylphenolpolyglykolether, Polyethylen-Rhizinusöl verwende werden.

Als phospholipidhaltige Gemische können alle Produkte eingesetzt werden, wobei der Phospholipidgehalt von 5—98% variieren kann, wie z.B. Lecithin-Naßschlämme, die je nach Herkunft folgende Zusammensetzung besitzen:

14—36% Pflanzenöle

27—56% Wasser

59— 8% Phospholipide

oder hochreine Phospholipide mit bis zu 98% Phosphatidylcholin.

Neben den Phospholipiden können in dem Gemisch alle von der Gewinnung der Phospholipide herrührenden Bestandteile vorhanden sein, wie z.B. Sterine, Öle, Zucker, Wasser, Glykolipide usw.

Die neuen Phospholipiderzeugnisse konnen als Zusätze für Pestizide und Düngemittel verwendet werden, insbesondere für Düngergranulat oder schuppenförmige Zubereitungen für die Nahrungsmittel, Futtermittel, Kosmetika und als Dispergiermittel für technische Zwecke.

Das neue Phospholipiderzeugnis kann auch als Träger für biologisch aktive Stoffe wie Arzneimittel, Pestizide, Nahrungsmittel, Dünger, Vitamine usw. verwendet werden.

Besonders bevorugt ist das Lösungsvermittlergemisch aus 1-Amino-2-hydroxy-ethan und 1-Hydroxy-2-trimethylamino-ethan-hydrochlorid im Gewichtsverhältnis von 1:5.

Der Phospholipidgehalt der wässrigen lösungen beträgt vorzugsweise 5—80 Gew.-%.

Beispiel 1

43,25 Gew.-% Sonnenblumen-Lecithin-Naßschlamm mit folgender Zusammensetzung:

16,2 Gew.-% Öl

52,2 Gew.-% Wasser

31,6 Gew.-% Phospholipide

werden in eine Lösung aus 1,0 Gew.-% 1-Amino-2-hydroxy-ethan und 5,0 Gew.-% 1-Hydroxy-2-trimethylamino-ethan-hydrochlorid sowie 1,0 Gew.-% Preventol® D 3 (Konservierungsmittel: synergistisch wirksame Mischung aus Arylmethanol und Halogenalkylacrylaminomethanol) und 49,75 Gew.-% Wasser eingerührt.

Die erhaltene Emulsion mit einem pH von ca. 8 und einer Viskosität von ca. 100 m Pas wurde mehrere Monate gelagert und war anwendungstechnisch stabil.

Beispiel 2

30 Gew.-% Soja-Lecithin mit folgender Zusammensetzung:

36 Gew.-% Sojaöl

64 Gew.-% Phospholipide

werden bei Raumtemperatur in eine Mischung, bestehend aus 1,0 Gew.-% 1-Amino-2-hydroxy-ethan, 1,5 Gew.-% Kokosamin ethoxyliert (Emulgator), 1,5 Gew.-% Alkylpolyglykolether (Emulgator), 0,54 Gew.-% Formalin, 5,0 Gew.-% 1-Hydroxy-2-trimethylamino-ethan-hydrochlorid und 60,46 Gew.-% Wasser eingerührt.

Die entstandene Formulierung mit einem pH von 9, einer Viskosität von <100 m Pas wurde einige Monate gelagert und war anwendungstechnisch stabil.

Beispiel 3

30 Gew.-% Raps-Lecithin werden bei Raumtemperatur in eine Mischung, bestehend aus 1,0 Gew.-%

Aminoalkanol von Beispiel 2, 1,5 Gew.-% Kokosamin ethoxyliert, 1,5 Gew.-% Alkylpolyglykolether, 5,0 Gew.-% 1-Hydroxy-2-trimethylamino-ethan-hydrochlorid, 0,54 Gew.-% Formalinlösung und 60,46 Gew.-% Wasser eingerührt.

Die entstandene Formulierung mit einem pH 8,5 und einer Viskosität <100 m Pas wurde mehrere Monate gelagert und war anwendungstechnisch stabil.

Beispiel 4

49,8 Gew.-% Sonnenblumen-Lecithin-Naßschlamm werden bei Raumtemperatur in eine Mischung, bestehend aus 1,0 Gew.-% Aminoalkanol von Beispiel 2, 3,0 Gew.-% Oxoalkohol ethoxyliert, 5,0 Gew.-% Trimethylamino-alkanol-phosphat, 0,54 Gew.-% Formalinlösung und 40,66 Gew.-% Wasser eingerührt. Die Formulierung wurde einige Wochen gelagert und war anwendungstechnisch stabil.

pH Wert 7, Viskosität 290 m Pas.

Beispiel 5

30 Gew.-% Sonnenblumen-Lecithin werden bei Raumtemperatur in eine Mischung, bestehend aus 1,0 Gew.-% Aminoalkanol von Beispiel 2, 1,0 Gew.-% Kokosamin ethoxyliert, 2,0 Gew.-% Oxoalkohol ethoxyliert, 0,5 Gew.-% Formalinlösung, 5 Gew.-% 1-Hydroxy-2-trimethylamino-ethan-hydrochlorid und 60,46 Gew.-% Wasser eingerührt.

Die erhaltene Formulierung mit einem pH 8,5 und einer Viskosität von ca. 200 m Pas wurde einige Monate gelagert und blieb anwendungstechnisch stabil.

Beispiel 6

51,4 Gew.-% Soja-Lecithin-Naßschlamm werden bei Raumtemperatur in eine Mischung, bestehend aus 1,0 Gew.-% Aminoalkanol von Beispiel 2, 2,0 Gew.-% Kokosamin ethoxyliert, 1,0 Gew.-% Alkylpolyglykolether, 5,0 Gew.-% Trimethylamino-alkanol-hydrochlorid, 0,54 Gew.-% Formalinlösung und 39,06 Gew.-% Wasser eingerührt. Viskosität <100 m Pas, pH 7. Die Formulierung war nach einigen Wochen anwendungstechnisch stabil.

Beispiel 7

20 Gew.-% Phospholipon$^R$ 25 werden in eine Lösung aus 1,0 Gew.-% 1-Amino-2-hydroxy-ethan und 5,0 Gew.-% 1-Hydroxy-2-trimethylamino-ethan-hydrochlorid sowie 1,0 Gew.-% Preventol D3 (Konservierungsmittel: synergistisch wirksame Mischung aus Arylmethanol und Halogenacrylaminoethanol) und 73 Gew.-% Wasser eingerührt. Die ethaltene Emulsion mit einem pH von ca. 8 und einer Viskosität von <100 mPas wurde mehrere Monate gelagert und war anwendungstechnisch stabil.

Beispiel 8

20 Gew.-% Phospholipon$^R$ 38 werden bei Raumtemperatur in eine Mischung, bestehend als 1,0 Gew.-% 1-Amino-2-hydroxy-ethan, 0,54 Gew.-% Formalin, 5,0 Gew.-% 1-Hydroxy-2-trimethylamino-ethan-hydrochlorid und 73,46 Gew.-% Wasser eingerührt.

Die entstandene Formulierung mit einem pH von 9, einer Viskosität <100 mPas wurde einige Monate gelagert und war anwendungstechnisch stabil.

Beispiel 9

20 Gew.-% Phospholipon$^R$ 80 werden bei Raumtemperatur in eine Mischung, bestehend aus 1,0 Gew.-% 1-Amino-2-hydroxy-ethan, 0,54 Gew.-% Formalin, 5,0 Gew.-% 1-Hydroxy-2-trimethylamino-ethan-hydrochlorid und 73,46 Gew.-% Wasser eingerührt.

Die entstandene Formlierung mit einem pH von 9, einer Viskosität von <100 mPas wurde einige Monate gelagert und war anwendungstechnisch stabil.

Beispiel 10

30 Gew.-% Phospholipon$^R$ 100 werden bei Raumtemperatur in eine Mischung, bestehend aus 1,0 Gew.-% 1-Amino-2-hydroxy-ethan, 0,54 Gew.-% Formalin, 5,0 Gew.-% 1-hydroxy-2-trimethylamino-ethan-hydrochlorid und 63,46 Gew.-% Wasser eingerührt.

Die entstandene Formulierung mit einem pH von 9, einer Viskosität <100 mPas wurde einige Monate gelagert und war anwendungstechnisch stabil.

Vergleich der Produkten, hergestellt ohne die in der vorliegenden Erfindungsmeldung genannten Verbindungen der Formel I bzw. mit diesen.

Vergleichsbeispiel 11

61,1 Gew.-% Soja-Lecithin-Naßschlamm, entsprechend 30 Gew.-% Soja-Lecithin, werden unter Rühren bei Raumtemperatur 1 Stunde lang mit 38,9 Gew.-% Wasser gemischt. Das erhaltene homogene, viskose Produkt besitzt eine Viskosität von 8000 m Pas, läßt sich mit Wasser nur durch intensives Rühren verdünnen und entspricht *nicht* den Anforderungen zur Herstellung von Spritzbrühen von Pflanzenbehandlungsmitteln.

Vergleichsbeispiel 12

30 Gew.-% Soja-Lecithin werden analog Beispiel 11 mit 70 Gew.-% Wasser gerührt. Das erhaltene Produkt besitzt eine Viskosität von 3400 m Pas und entspricht ebenfalls *nicht* den Anforderungen zur Herstellung von Spritzbrühen von Pflanzenbehandlungsmitteln.

Vergleichsbeispiel 13

20 Gew.-% Phospholipon$^R$ 80 werden analog Beispiel 11 mit 80% Wasser gemischt. Das erhaltene Produkt besitzt eine Viskosität von ca. 17000 mPas und läßt sich nur mit Hilfe eines Rührers oder Mischers mit Wasser verdünnen.

Vergleichsbeispiel 14

30 Gew.-% Phospholipon$^R$ 100 werden analog Vergleichsbeispiel 11 mit 70 Gew.-% Wasser gemischt. Das erhaltene Produkt besitzt eine Viskosität von ca. 7000 mPas und läßt sich nur mit Hilfe eines Rührers oder Mischers mit Wasser verdünnen.

| Zusammensetzung nach | Phospholipidkonzentration | Viskosität in mPas | Aussehen der 10%igen Formulierungsdispersion *ohne* mechanische Hilfe | Kompatibilität der Formulierungsdispersion mit | | |
|---|---|---|---|---|---|---|
| | | | | Herbizid-Spritzbrühen[1)] | Fungizid-Spritzbrühen[2)] | Blattdünger-Applikationslösungen[3)] |
| Beispiel 9 | 20% Phospholipon[R] 80 | 100 | homogen | + | + | + |
| Vergleichsbeispiel 13 | | 17000 | inhomogen, klumpig | – | – | – |
| Beispiel 10 | 30% Phospholipon[R] 100 | 100 | homogen | + | + | + |
| Vergleichsbeispiel 14 | | 7000 | inhomogen, klumpig | – | – | – |
| Beispiel 6 | 30% Soja-Lecithin-Naßschlamm | 100 | homogen | + | + | + |
| Vergleichsbeispiel 11 | | 8000 | inhomogen, klumpig | – | – | – |
| Beispiel 2 | 30% Soja-Lecithin | 100 | homogen | + | + | + |
| Vergleichsbeispiel 1 | | 3400 | inhomogen, klumpig | – | – | – |
| Beispiel 7 | 20% Phospholipon[R] 25 | 100 | homogen | + | + | + |
| Beispiel 8 | 20% Phospholipon[R] 38 | 100 | homogen | + | + | + |

+=Die Formulierungsdispersion in Wasser ist mit der Spritzbrühe oder der Applikationslösung kompatibel

–=Die Formulierungsdispersion in Wasser ist mit der Spritzbrühe oder der Applikationslösung nicht kompatibel

Zu 1)

Bei den Herbizid-Spritzbrühen wurden u.a. folgende Wirkstoffe in Form von Handelsprodukten geprüft:

Atrazin
Isoproturon

Zu 2)

Bei den Fungizid-Spritzbrühen wurden u.a. folgende Wirkstoffe in Form von Handelsprodukten geprüft:

Folpet
Procymidon
Chlorthelonil

Zu 3)

Es wurden u.a. Blattdünger mit folgender Zusammensetzung geprüft:

| | Typ A | Typ B |
|---|---|---|
| N | 20 | 8 |
| $P_2O_5$ | 10 | 8 |
| $K_2O$ | 15 | 6 |
| MgO | 4 | 0,01 |
| Fe | 0,4 | 0,01 |
| Zn | 0,1 | 0,01 |
| Mn | 0,15 | 0,01 |
| andere Spurenelemente | 0,1 | 0,025 |

Vergleicht man die Beispiele 1—10, hergestellt entsprechend den erfindungsgemäßen Zusammensetzungen mit den Vergleichsbeispielen 11—14, hergestellt ohne den erfindungsgemäßen Zusatz von Verbindungen der Formel I, so laßt sich anhand der vorhergehenden Tabelle eindeutig feststellen, daß nur die erfindungsgemäß hergestellten Produkte dem Anspruch einer beliebigen, durch einfaches Mischen ohne mechanische Hilfe herstellbaren Verdünnung mit Wasser genügen.

Weiterhin läßt sich feststellen, daß nur durch die erfindungsgemäß hergestellten Formulierungen der Anspruch der Kompatibilität mit Herbizid/Fungizid Spritzbrühen oder Blattdünger-Applikationslösungen erfüllt wird.

Beispiel 15

Eine Spritzbrühe, entsprechend 3,0 Liter Handelsprodukt (a.i.=Isoproturon) pro ha, wird durch Verdünnen mit Wasser hergestellt und ihre Oberflächenspannung mittels Tensiomat® gleich 51,8 mN/m gemessen. Durch Zugabe von Produkt gleich Beispiel 9 in einer Menge von 4 kg/ha wird die Oberflächenspannung auf 39 mN/m reduziert und dadurch eine wesentlich bessere Benetzung der Blattoberfläche nach der Applikation der Spritzbrühe erreicht.

Nachdem zur Herstellung der Spritzbrühen von Pflanzenbehandlungsmitteln nur geringe mechanische Kräfte zur Verfügung stehen, müssen sich die zur Spritzbrühe zu verarbeitenden Produkte, gleich ob fest oder flüssig, praktisch spontan in Wasser verteilen bzw. emulgieren. Das Vermischen der Produkte mit Wasser wird lediglich durch Umpumpen herbeigeführt.

Beispiel 16

In nachfolgender Tabelle sind die Ergebnisse biologischer Prüfungen unter Gewächshausbedingungen ermittelt dargestellt. Hierbei wurden die als Handelsprodukte formulierten Wirkstoffe in Form von Spritzbrühen (A) bzw. in Form von Tankmischungen (B) appliziert.

Unter Tankmischungen (B) sind Spritzbrühen zu verstehen, die hergestellt werden durch Verdünnen eines Handelsproduktes mit Wasser und Zugabe eines im vorliegenden Fall erfindungsgemäß hergestellten Produktes auf der Basis von Lecithin-Naßschlamm.

Die Ergebnisse biologischer Herbizid-Prüfungen sind dargestellt als prozentuelle Schädigung der mit Spritzbrühen oder Tankmischungen behandelten Pflanzen (Unkräuter).

0% bedeutet keine Schädigung, d.h., unwirksam 100% bedeutet totale Schädigung, d.h. maximale Wirksamkeit.

Es sind jeweils zu vergleichen die Versuche a und b derselben Ziffer.

Die Zweckmäßigkeit des Zusatzes der erfindungsgemäß hergestellten Beispiele ist in den höheren Schädigungsprozenten verdeutlicht.

Im Falle der vorliegenden biologischen Prüfung mit Wachstumsregulatoren wird die Höhe der Halme in cm gemessen.

Die Halmverkürzung ist ein erwünschter Effekt.

Im Falle der beschriebenen biologischen Prüfung mit Fungiziden werden Befallsstärke (BS) und Wirkungsgrad (WG) bei der Bekämpfung von Botrytis im Weinbau ermittel. Je niedriger die Befallsstärke und je höher der Wirkungsgrad ermittelt werden, umso wirksamer ist die Fungizid-Behandlung.

Biologische Prüfungen — Beispiel 12

| | Handelsprodukt (HP) | | Spritzbrühentype | | Schädigung der Unkräuter in % | | | | |
|---|---|---|---|---|---|---|---|---|---|
| Nr. | Wirkstoff | HP pro Hektar | A | B | Sinapis alba | Poa annua | Panicum miliaceum | Solanum lycopersicum | Alopecurus myosaroide |
| 0 | 1 | 2 | 3 | 4 | 5 | 6 | 7 | 8 | 9 |
| 1a | Isoproturon | 0,700 kg | HP | | 35 | | 30 | 20 | 30 |
| 1b | Isoproturon | 0,700 kg | | HP+ Beisp. 3 | 67 | | 75 | 57 | 58 |
| 2a | Isoproturon | 0,700 kg | HP | | 40 | | 27 | 30 | 45 |
| 2b | Isoproturon | 0,700 kg | | HP+ Beisp. 5 | 81 | | 50 | 37 | 60 |
| 3a | Isoproturon | 1,000 kg | HP | | 40 | | 33 | 30 | 50 |
| 3b | Isoproturon | 1,000 kg | | HP+ Beisp. 6 | 70 | | 73 | 85 | 60 |
| 4a | Metamitron | 2,000 kg | HP | | 45 | | 20 | 35 | 31 |
| 4b | Metamitron | 2,000 kg | | HP+ Beisp. 3 | 55 | | 30 | 45 | 40 |
| 5a | Metamitron | 2,000 kg | HP | | 52 | | 0 | 40 | |
| 5b | Metamitron | 2,000 kg | | HP+ Beisp. 1 | 75 | | 10 | 60 | |
| 6a | Atrazin | 1,875 kg | HP | | 50 | 20 | 10 | 45 | |
| 6b | Atrazin | 1,875 kg | | HP+ Beisp. 3 | 80 | 35 | 15 | 88 | |

| | Handelsprodukt (HP) | | Spritzbrühentype | | Schädigung der Unkräuter in % | | | | |
|---|---|---|---|---|---|---|---|---|---|
| Nr. | Wirkstoff | HP pro Hektar | A | B | Sinapis alba | Poa annua | Panicum miliaceum | Solanum lycopersicum | Alopecurus myosaroide |
| 0 | 1 | 2 | 3 | 4 | 5 | 6 | 7 | 8 | 9 |
| 7a | Atrazin | 2,000 kg | HP | | 50 | | 8 | | |
| 7b | | 2,000 kg | | HP+ Beisp. 4 | 75 | | 10 | | |
| 8a | Atrazin | 1,875 kg | HP | | 50 | 20 | 10 | 40 | |
| 8b | | 1,875 kg | | HP+ Beisp. 2 | 93 | 36 | 13 | 85 | |
| 9a | Diuron | 2,000 kg | HP | | 59 | | | 14 | 15 |
| 9b | | 2,000 kg | | HP+ Beisp. 1 | 84 | | | 58 | 43 |

Versuche mit Wachstumsregulatoren

Testpflanze: Winterweizen

| Pos. | Behandlung | Aufwandmenge in Liter/ha | Höhe der Halme in cm |
|---|---|---|---|
| 1. | Kontrolle (unbehandelt) | — | 44 |
| 2. | Etophon/Mepiquat-chlorid Handelsprodukt | 2,5 | 38 |
| 3. | Pos. 2+Beispiel 18 | 2,5+2 | 25 |

Versuche zur Botrytisbekämpfung

Testpflanze: Weinrebe, Sorte Müller-Thurgau

| Pos. | Behandlung | Konzentration in der Spritzbrühe | BS % | WS % |
|---|---|---|---|---|
| 1. | Kontrolle | Beispiel 8, 0,4% | 35 | 0 |
| 2. | Folpet-Handelsprodukt | 0,15 | 16 | 51 |
| 3. | Folpet-Handelsprodukt + Beispiel 8 | 0,15<br>0,4 | 8 | 74 |

BS=Befallsstärke
WS=Wirkungsgrad

Beispiel 17

30 Gew.-% Soja-Lecithin werden bei Raumtemperatur in eine Mischung aus 1,0 Gew.-% 1-Amino-2-hydroxy-alkan, 4,5 Gew.-% Kokosamin ethoxyliert, 4,5 Gew.-% Alkylpolyglykolether, 7 Gew.-% 70 %iges 1-Hydroxy-2-trimethylammonium-chlorid-alkan und 53 Gew.-% Wasser eingerührt. Die erhaltene Formulierung hat eine Viskosität von 200 m Pas und war mehrere Monate anwendungstechnisch stabil.

Beispiel 18

30 Gew.-% Soja-Lecithin werden bei Raumtemperatur in eine Mischung aus 1,0 Gew.-% 1-Amino-2-hydroxy-alkan, 7,5 Gew.-% Kokosamin ethoxyliert, 7,5 Gew.-% Arylalkylpolyglykolether, 7 Gew.-% 70 %iges 1-Hydroxy-2-trimethl-ammoniumchlorid-alkan und 47 Gew.-% Wasser eingerührt. Die erhaltene Formulierung hat eine Viskosität von 180 m Pas und war über mehrere Monate anwendungsstabil.

Beispiel 19

30 Gew.-% Soja-Lecithin werden bei Raumtemperatur in eine Mischung aus 1,0 Gew.-% 1-Amino-2-hydroxy-alkan, 7 Gew.-% 70 %iges 1-Hydroxy-2-trimethylammoniumchlorid-alkan, 15 Gew.-% Fettalkohol ethoxyliert und 47 Gew.-% Wasser eingerührt. Die erhaltene Formulierung hat eine Viskosität von 120 m Pas und war über mehrere Monate anwendungsstabil.

Beispiel 20

10 kg lecithinhaltiges Produkt, hergestellt analog Beispiel 2 (Konservierungsmittel K. sorbat+Natr. benzoat) werden geknetet mit 100 kg Weizenmehl Typ 550, 4 kg Hefe, 2 kg Salz, 1 kg Erdnußfett, 1 kg Zucker, 3 g Ascorbinsäure, 400 g Calciumacetat, 50 kg Wasser. Temperatur der Knetmasse 30°C, Knetzeit 2 Minuten. Die erhaltene Masse wird 15 Minuten ruhen gelassen. Nach einer Zwischengärzeit von 10 Minuten und einer Endgärzeit von 90 Minuten erhält man ein hochwertiges Backmittel.

Beispiel 21

2 kg lecithinhaltiges Produkt, hergestellt nach Beispiel 2, werden mit 13 kg Zinkoxyd, 11 kg Titandioxyd, 5,5 kg Kreide, 5 kg Kaolin, 200 g Natr.-Kal. Hexametaphosphat, 4,3 kg Stabilisierungsmittel, 19 kg Wasser und 39 kg Polyvinylester zu einer sterilen Dispersion gemischt.

Beispiel 22

Im Verhältnis 0,8—2 zu 1000 wird lecithinhaltiges Produkt, gefertigt nach Beispiel 2, konserviert mit K.

sorbat und Natr. benzoat, auf getrocknete Milch aufgesprüht. Das erhaltene Vollmilchpulver besitzt gute Instanteigenschaften.

Beispiel 23

5 kg Rohkaffee werden in einem Röstgerät auf 220°C erwärmt. Nach Erreichen dieser Temperatur sprüht man ein lecithinhaltiges Produkt, hergestellt nach Beispiel 9, (Konservierungsmittel Natr. benzoat+Kal. sorbat) in einer Menge auf, so daß die getrockneten gerösteten Kaffeebohnen mit ca. 10 g Lecithin pro kg gerösteten Kaffeebohnen überzogen sind.

Die hierdurch mit einem Aromaschutz versehenen Kaffeebohnen können in üblicher Weise vermahlen und zu Kaffeegetränken verarbeitet werden.

Beispiel 24

10,6 kg Weizenkeimöl, 1,7 kg Bienenwachs, 1,7 kg Oleum Cacao DAB 8, 1,2 kg Cetylstearylalkohol DAB 8, 2,1 kg Wollwachs DAB 8, 0,8 kg Polyoxyethylen-sorbitanmonooleat und 0,08 kg Benzoesäure werden bei 70°C geschmolzen und gemischt. 16,6 kg lecithinhaltiges Produkt, hergestellt nach Beispiel 10, werden langsam eingerührt und mit 22 kg Wasser nachfolgend ergänzt. Die erhaltene Masse wird unter Rühren abgekühlt und nach Erreichen der Raumtemperatur homogenisiert. Das Produkt, gefertigt nach Beispiel 26, kann als kosmetische Feuchtigkeitscreme verwendet werden.

**Patentansprüche**

1. Wässrige Phospholipidlösungen, die Lösungsvermittler enthalten, dadurch gekennzeichnet, daß als Lösungsvermittler ein oder mehrere Verbindungen der Formel (I)

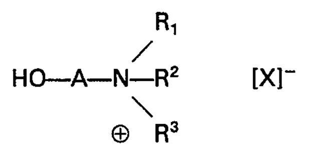

I

in der A eine gradkettige Alkylengruppe mit 2 bis 4 Kohlenstoffatomen, $R^1$, $R^2$, $R^3$ gleich oder verschieden sein können und Wasserstoff oder Methyl bedeuten und X das zur Salzbildung dienende Anion einer anorganischen oder organischen Säure ist, und/oder 1-Methylamino-2-hydroxy-ethan, 1-Dimethylamino-2-hydroxy-ethan, 1-Dimethylamino-2-hydroxy-butan, 1-Hydroxy-2-methyl-amino-hexan, 1-Amino-2-hydroxy-ethan, 1-Amino-3-hydroxy-propan, 1-Amino-2-hydroxy-propan, 1-Dimethylamino-2-hydroxy-propan, 1-Amino-3-hydroxy-butan vorhanden sind.

2. Wässrige Phospholipidlösungen gemäß Anspruch 1, dadurch gekennzeichnet, daß als Lösungsvermittler ein Gemisch aus 1-Amino-2-hydroxy-ethan und 1-Hydroxy-2-trimethylamino-ethan-hydrochlorid eingesetzt wird.

3. Wässrige Phospholipidlösungen gemäß Anspruch 2, dadurch gekennzeichnet, daß das Lösungsvermittlergemisch aus 1-Amino-2-hydroxy-ethan und 1-Hydroxy-2-trimethylamino-ethan-hydrochlorid im Gewichtsverhältnis von 1:5 eingesetzt wird.

4. Wässrige Phospholipidlösungen gemäß einem oder mehreren der Ansprüche 1 bis 3, dadurch gekennzeichnet, daß man 3 bis 10 Gew.% des Lösungsvermittlers bzw. des Lösungsvermittlergemisches einsetzt.

5. Wässrige Phospholipidlösungen gemäß einem oder mehreren der Ansprüche 1 bis 3, dadurch gekennzeichnet, daß man 5 bis 7 Gew.% des Lösungsvermittlers bzw. des Lösungsvermittlergemisches einsetzt.

6. Wässrige Phospholipidlösungen gemäß einem oder mehreren der Ansprüche 1 bis 3, dadurch gekennzeichnet, daß man 6 Gew.% des Lösungsvermittlers bzw. des Lösungsvermittlergemisches einsetzt.

7. Wässrige Phospholipidlösungen gemäß einem oder mehreren der Ansprüche 1 bis 6, dadurch gekennzeichnet, daß der Phospholipidgehalt in der Lösung 5 bis 80 Gew.% beträgt.

**Revendications**

1. Solutions aqueuses de phospholipides contenant des agents de dissolution, caractérisées, en ce qu'il est présent, comme agents de dissolution un ou plusieurs composés de formule (I)

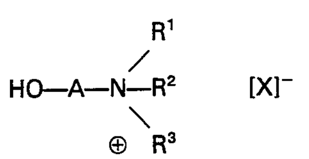

I

dans laquelle A est une groupe alcoyle à chaîne droite avec de 2 à 4 atomes de carbone, $R^1$, $R^2$, $R^3$ peuvent être identiques ou différentes et représentent de l'hydrogène ou un radical méthyle et X est l'anion servant à la formation de sel d'un acide inorganique ou organique, et/ou le 1-méthylamino-2-hydroxy-éthane, le 1-diméthylamino-2-hydroxy-éthane, le 1-diméthylamino-2-hydro-butane, le 1-hydroxy-2-méthylamino-hexane, le 1-amino-2-hydroxy-éthane, le 1-amino-3-hydroxy-propane, le 1-amino-2-hydroxy-propane, le 1-diméthylamino-2-hydroxy-propane, le 1-amino-3-hydroxy-butane.

2. Solutions aqueuses de phospholipides suivant la revendication 1, caractérisées en ce qu'il est utilisé, comme agent de dissolution, un mélange de 1-amino-2-hydroxy-éthane et de chlorure de 1-hydroxy-2-triméthylamino-éthane.

3. Solutions aqueuses de phospholipides suivant la revendication 2, caractérisées en ce que le mélange d'agents de dissolution constitué de 1-amino-2-hydroxy-éthane et de chlorure de 1-hydroxy-2-triméthyl-amino-éthane est utilisé dans un rapport en poids de 1:5.

4. Solutions aqueuses de phospholipides suivant l'une ou plusieurs des revendications 1 à 3, caractérisées en ce que l'on utilise de 3 à 10% en poids d'agent de dissolution ou du mélange d'agents de dissolution.

5. Solutions aqueuses de phospholipides suivant l'une ou plusieurs des revendications 1 à 3, caractérisées en ce que l'on utilise de 5 à 7% en poids d'agent de dissolution ou du mélange d'agents de dissolution.

6. Solutions aqueuses de phospholipides suivant l'une ou plusieurs des revendications 1 à 3, caractérisées en ce que l'on utilise 6% en poids d'agent de dissolution ou du mélange d'agents de dissolution.

7. Solutions aqueuses de phospholipides suivant l'une ou plusieurs des revendications 1 à 6, caractérisées en ce que la teneur en phospholipides de la solution est de 5 à 80% en poids.

**Claims**

1. Aqueous phospholipid solutions containing a solubilizing agent, characterized in that one or more compounds of the formula (I)

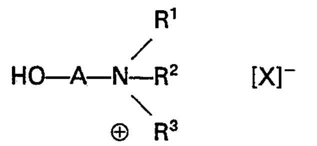

I

are present as solubilizing agent, where A is a straight chain alkylene group having 2 to 4 carbon atoms, $R^1$, $R^2$ and $R^3$ can be identical or different and denote hydrogen or methyl, and X is the anion of an inorganic or organic acid effecting the salt formation, and/or 1-methylamino-2-hydroxy-ethane, 1-dimethylamino-2-hydroxy-ethane, 1-dimethylamino-2-hydroxy-butane, 1-amino-3-hydroxy-propane, 1-amino-2-hydroxy-propane, 1-dimethylamino-2-hydroxy-propane, 1-amino-3-hydroxy-butane.

2. Aqueous phospholipid solutions according to claim 1, characterized in that a mixture of 1-amino-2-hydroxy-ethane and 1-hydroxy-2-trimethylamino-ethane-hydrochloride are employed as solubilizing agent.

3. Aqueous phospholipid solutions according to claim 2, characterized in that the mixture of solubilizing agent from 1-amino-2-hydroxy-ethane and 1-hydroxy-2-trimethylamino-ethane-hydrochloride is employed at a weight ratio of 1:5.

4. Aqueous phospholipid solutions according to one or more of the claims 1 to 3, characterized in that 3 to 10 weight percent of the solubilizing agent resp. the mixture thereof are employed.

5. Aqueous phospholipid solutions according to one or more of the claims 1 to 3, characterized in that 5 to 7 weight percent of the solubilizing agent resp. the mixture thereof are employed.

6. Aqueous phospholipid solutions according to one or more of the claims 1 to 3, characterized in that 6 weight percent of the solubilizing agent resp. the mixture thereof are employed.

7. Aqueous phospholipid solutions according to one or more of the claims 1 to 6, characterized in that the phospholipid content in the solution is 5 to 80 weight percent.